# EUROPEAN PATENT SPECIFICATION

(11) **EP 0 393 147 B1**
(45) Date of publication and mention of the grant of the patent: **31.05.1995**
(21) Application number: 89901702.4
(22) Date of filing: 19.12.1988
(51) Int. Cl.: C12P 7/44, C12P 7/20, C12P 7/06, C12F 3/10

(54) **PROCESS FOR MANUFACTURING ETHANOL, GLYCEROL AND SUCCINIC ACID**
VERFAHREN ZUR PRODUKTION VON ETHANOL, GLYCERIN UND BERNSTEINSÄURE
PROCEDE DE PRODUCTION D'ETHANOL, DE GLYCEROL ET D'ACIDE SUCCINIQUE

(30) Priority: 22.12.1987 US 136415
(43) Date of publication of application: 24.10.1990
(73) Proprietor: KAMPEN, Willem Hemmo, Charlotte, NC 28213 (US)
(72) Inventor: KAMPEN, Willem Hemmo, Charlotte, NC 28213 (US)
(74) Representative: Warren, Keith Stanley
(86) International application number: PCT/US88/04529
(87) International publication number: WO 89/05861

(56) References cited:
- DE-B- 1 063 559
- JP-A- 4 858 191
- US-A- 2 772 206
- US-A- 2 772 207
- US-A- 4 287 305
- B. BURRIS, "Recovery of Chemicals Such as Glycerol, Dextrose, and Amino Acids from Dilute Broths", presented to International Conference on Fuel, Alcohols and Chemicals from Biomass, 11-12 November 1986, Miami Beach, FL (US)/
- B. BURRIS, "Recovery of Glycerol from Stillbottoms", presented to International Conference on Fuel, Alcohols and Chemicals from Biomass, 07-09 December 1987, Montego Bay, Jamaica/

## Description

This invention relates to the manufacture of ethanol, recovering glycerol as a by-product. The process may also be used to recover succinic acid and a free flowing distiller's dry grain and solubles useful as an animal feed.

The manufacture of all of the products named has been known heretofore, and all have commercial uses. Ethanol is used as a beverage, a chemical, and a fuel derived from renewable resources, and is typically manufactured by fermentation and distillation processes starting from biological materials such as grain or the like. Glycerol, while known to be produced as a by-product of ethanol fermentation and distillation processes, has been manufactured commercially by processes which have soap as the primary product or which synthesize glycerol from petrochemical feedstocks. Succinic acid is synthesized from maleic or acetic acid. Distiller's dry grain, characterized as with or without solubles, is conventionally produced as a by product of fermentation and distillation processes, and is usually sufficiently infused with sticky by products such as glycerol as to have poor flowing qualities and be difficult to handle.

The manufacture of ethanol is sufficiently well known that the interested reader is referred to the available literature for descriptions of the basic processes. The manufacture of glycerol is, by way of example, discussed in Hildebrandt United States Patent 2,160,245; Wallerstein United States Patent 2,400,859; and Frankel United States Patent 2,772,207, to which the interested reader is referred. The manufacture of succinic acid and free flowing distiller's dry grain by processes such as those to be described hereinafter has not, insofar as is known to the present inventor, been described in any prior patent.

Brian D. Burris, in a paper presented to the International Conference on Fuel Alcohols and Chemicals from Biomass in Miami in 1986, disclosed a process for recovering glycerol and other components from dilute fermentation broths by a combination of ultrafiltration and ion exchange techniques. At the same Conference the following year at Montego Bay, Jamaica, he disclosed various processes, including ion exclusion, for recovering glycerol from stillbottoms.

The present invention consists in a process for manufacturing ethanol and recovering glycerol as a by-product thereof, comprising the steps of:
Preparing a biomass mash;
fermenting the mash with yeast;
distilling the fermented mash to produce ethanol and stillage containing glycerol;
clarifying the stillage; and
passing the clarified stillage through an ion exclusion material for separating glycerol from the other constituents of the clarified stillage, characterised in that the stillage is clarified by subjecting it to a cross-flow microfiltration system, having ceramic or mineral membranes, which is able to separate particles in the size range 0.1 to 10 »m.

By means of the present invention, the efficiency of the known ethanol producing fermentation and distillation processes is essentially maintained, while an additional valuable product is derived.

A feature of preferred embodiments of this invention is the manufacture of succinic acid as a by-product of a fermentation. In realizing this object of the invention, succinic acid is produced by an essentially natural biochemical process, starting from a biologically based material and without synthesis of the type used heretofore.

A further feature of preferred embodiments of the invention is the manufacture of free flowing distiller's dry grain. Distiller's dry grain is a known by-product of fermentation and distillation processes, useful as an animal feed. However, the handling of distiller's dry grain (known as DDG or DDGS depending upon the presence of solubles) has been made difficult heretofore by the presence of small quantities of glycerol and the like which render the product less flowable. In preferred processes according to the present invention, DDG or DDGS becomes handleable using conventional procedures for free flowing dry materials, thus increasing the value of the by-product.

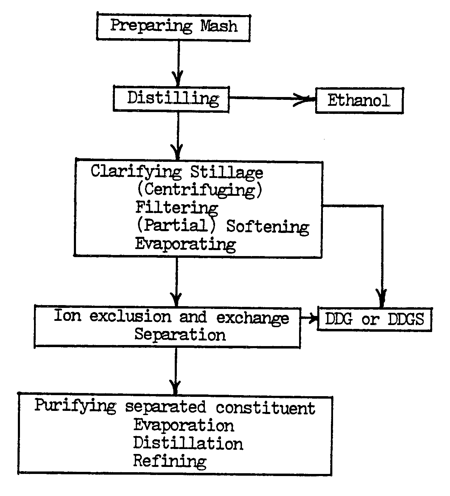

Preferred embodiments of the invention will now be described with reference to the accompanying drawing, in which a schematic representation of the flow of materials in the processes and apparatus of the present invention is shown.

Referring now more particularly to the drawing, this represents certain steps and apparatus which, in the sequence disclosed hereinafter, accomplish the objects of this invention. Certain steps and apparatus, being well known to those having skill in the relevant arts, have not been shown but will be described for the reader.

It is known that the formation of ethanol in a fermentation process is growth associated and that the formation of glycerol and succinic acid is interrelated. In a typical batch fermentation process without the recycle of stillage, some 46.5 g of ethanol, 3.5g of glycerol and 0.6 g of succinic acid are formed per 100 grams of reducing sugar consumed. The present invention at least in its preferred embodiments, realises the commercially feasible recovery of the glycerol and succinic acid constituents, resulting in the production of free flowing DDG and/or DDGS, and contemplates the enhancement of the production of the by products to be recovered by the adjustment of several process parameters. These process parameters and the result of operation in accordance with the present invention will be addressed hereinafter.

The first process parameter to be addressed relates to the form of yeast used in the fermentation process. It has been determined that a properly immobilized organism will enhance and increase production of glycerol and succinic acid. These results are achieved where the yeast is in a stable ionic and high density matrix, as illustrated in the following example.

### Example 1

A mash prepared of ground whole corn was subjected to liquefaction at a pH of 6.3 to a dextrose equivalent (DE) of 20.3; to jet cooking for two minutes at 149 degrees Celsius; and to saccharification to DE 36 at pH 4.5. Immobilized yeast cells were prepared by mixing a 1.5 weight percent sodium alginate solution with the preferred ratio of hydrated yeast cells and sterilized sand. The resulting slurry was poured through a 12 mesh screen into an aqueous solution of 0.5 M CaCl₂ and 1.5 weight percent glucose at pH 4.6 and ambient temperature. On contact with the calcium chloride, the drops formed beads which, after 24 hours in a refrigerator at 4 degrees Celsius, gelatinized into firm beads with diameters of 2 - 4 mm. The mash was then fermented in two batches at 34 degrees Celsius with free and immobilized yeast cells at concentrations of 5.0 grams per liter. Fermentation was performed in a batch process with no recycling of stillage and adjustment of pH to 5.0 using NaOH. Comparing the two batches, yields were as follows:

| Yeast | Glycerol g/100g | Succinic Acid Reducing Sugar (RS) |
|---|---|---|
| Free Cells | 3.38 | 0.67 |
| Immobilized | 4.09 | 0.87 |

Other process parameters which can be adjusted to optimize the process of this invention include yeast cell concentration and DE or carbohydrate concentration. The yeast cell concentration is preferably in excess of 300 x 10⁶ per ml. As yeast cell concentration and carbohydrate concentration rise, production of glycerol and succinic acid are enhanced. These characteristics are illustrated in the following example.

The mash is preferably prepared with a dextrose equivalent (DE) of at least 30 and a reducing sugar concentration of at least 200g/l.

### EXAMPLE 2

A mash was prepared from ground whole milo and fermentation started at pH 4.9 and DE 27 using free yeast cells and at a temperature of 33°C. Fermentation was performed in batch mode, without stillage recycle. Yields, related to yeast cell and carbohydrate concentration, were as follows:

| Yeast cell concentration x 10⁶ml | DE | Glycerol g/100g | Succinic Acid RS |
|---|---|---|---|
| 100 | 27 | 3.03 | 0.49 |
| 300 | 27 | 3.07 | 0.52 |
| 500 | 27 | 3.19 | 0.61 |
| 500 | 46 | 3.37 | 0.64 |
| 1,500 | 90 | 5.01 | 0.83 |

These results reflect enhanced glycerol and succincic acid production following from increased concentration of yeast cells and fermentable carbohydrates during fermentation.

Still other process variables which are contemplated by this invention as being optimized for the production of the desired by products while maintaining ethanol production include osmotic pressure during fermentation; concentration of dissolved carbon dioxide; pH; temperature; selection of the microorganism used; the mode of fermentation; and the formulation and preparation of fermentation media. More precisely, increased osmotic pressure from recycling stillage, increased solute concentration, and/or increased temperature increases production of glycerol and succinic acid, as does increased dissolved carbon dioxide. Yeast cells, as most microorganisms, are able to regulate its internal pH quite effectively within a range of mash pH of from 3 to 7, and thus the effect of process variations in pH may appear minimal. It has been determined, however, that production of glycerol and succinic acid is enhanced where an essentially constant pH is maintained during the first half of the fermentation step (when the bulk of glycerol and succinic acid are produced) by the addition of a suitable alkali such as sodium carbonate. The fermentation media, or mash, characteristics will affect the production of glycerol and succinic acid due to the interacting effects of the raw material itself, the concentration of any liquefying (metallo) enzyme, the ratio of fermentable sugars to nonsugars, the type of nonsugars present, and the nutritional requirements of the particular fermentation microorganism chosen.

Proper selection of the parameters described above will result in enhanced production of glycerol, and preferably succinic acid, as contemplated by this invention. The following examples include illustrations of variation in the various parameters and the effects on glycerol and succinic acid production of those variations.

### Example 3

A mash prepared from ground whole corn was liquefied to DE 20.6 and subjected to jet cooking for 3 minutes at 152°c and partial saccharification. Batches of the mash were then fermented and distilled using process parameters as set forth in the following table, with yields as noted.

| Process Parameter | Batch | | | |
|---|---|---|---|---|
| | A | B | C | D |
| YCC | 1.2 | 3.1 | 9.0 | 26.5 |
| DE | 33.2 | 56.3 | 78.7 | 78.7 |
| Recycle | 0 | 38.3 | 44.1 | 71.4 |
| Temperature | 30 | 34 | 35 | 35 |
| pH | 4.5 | 5.5 | 6.0 | 5.0 |
| Head (psig) | atm. | 1.7 | 2.3 | 1.2 |
| Head (kPa) | atm. | 11.7 | 15.9 | 8.3 |
| Yeast type | free | immob. | immob. | immob. |
| Time | 58 | 39 | 12 | 9 |

| Yields grams/100 grams RS | | | | |
|---|---|---|---|---|
| Ethanol | 44.9 | 44.1 | 42.0 | 44.7 |
| Glycerol | 4.8 | 5.8 | 8.3 | 12.3 |
| Succinic acid | 0.6 | 0.7 | 1.1 | 1.4 |

In the table, YCC refers to yeast cell concentration in grams per liter with approximately 10¹⁰ cells per gram. DE refers to dextrose equivalent after saccharification. Recycle refers to the percentage of recycled stillage in the mash being processed. Temperature is the temperature of fermentation in degrees Celsius. The reference to pH is to the value at which pH was maintained during the first half of the fermentation by the addition of sodium carbonate. The "Head" was head pressure. Time is in hours for fermentation.

Considering the tabulated examples together, it is noted that production of glycerol and succinic acid was substantially enhanced with little adverse affect on ethanol production.

### Example 4

A mash was prepared from clarified and pasteurized blackstrap molasses, and batches of the mash were then fermented and distilled using process parameters as set forth in the following table, with yields as noted.

| Process Parameter | Batch | | | | |
|---|---|---|---|---|---|
| | A | B | C | D | E |
| YCC | 1.0 | 3.0 | 18.2 | 32.0 | 20 |
| RS | 184 | 184 | 192 | 200 | 200 |
| Recycle | 0 | 24.7 | 43.0 | 37.9 | 48.4 |
| Temperature | 30 | 34 | 34 | 35 | 35 |
| Head (psig) | atm. | 0.9 | 1.8 | 2.1 | 1.2 |
| Head (kPa) | atm. | 6.2 | 12.4 | 14.5 | 8.3 |
| Yeast type | free | free | immob. | immob. | immob. |
| Fermentation | batch | batch | batch | cont. | batch |
| Time | 51 | 27 | 10 | 6 | 12 |
| pH | 4.5 | 5.0 | 6.0 | 5.5 | 5.0 |

| Yields grams/100 grams RS | | | | | |
|---|---|---|---|---|---|
| Ethanol | 48.3 | 47.8 | 43.9 | 46.4 | 45.1 |
| Glycerol | 3.7 | 4.3 | 8.4 | 5.1 | 10.9 |
| Succinic acid | 0.5 | 0.6 | 1.0 | 0.8 | 1.2 |

In the table, RS refers to reducing sugar concentration in grams per liter and "Fermentation" refers to a choice between batch and continuous processes, while the other parameters are as identified above in the description of Example 3.

It will be observed that parameters may be found at which the production of glycerol and succinic acid drop below the maximized levels. However, the maximized levels of production of the by-products sought by the present invention is attained without significant impairment of ethanol production.

### Example 5

In order to illustrate other processes without characterizing the additional processes as achieving optimal production of glycerol and succinic acid, a clarified wood hydrolyzate was prepared from yellow pine and processed by batch fermentation with pH held constant for 25 hours, without recycling of stillage and with parameters and yields as follows:

| Process Parameter | Batch | | |
|---|---|---|---|
| | A | B | C |
| YCC | 15.0 | 40.0 | 40.0 |
| RS | 54.3 | 54.3 | 74.1 |
| Temperature | 31 | 33 | 34 |
| Head (psig) | atm. | 0.3 | 1.2 |
| Head (kPa) | atm. | 2.1 | 8.3 |
| Yeast type | free | immob. | immob. |
| Fermentation | batch | batch | batch |
| Time | 68 | 43 | 41 |
| pH | 5.0 | 5.5 | 5.0 |

| Yields grams/100 grams RS | | | |
|---|---|---|---|
| Ethanol | 29.4 | 34.5 | 40.4 |
| Glycerol | 3.4 | 3.9 | 6.9 |
| Succinic acid | 0.5 | 0.8 | 1.1 |

In accordance with this invention, a fermented mash prepared in accordance with any of the above examples is then further processed to yield pure glycerol derived from natural sources (and thus of kosher purity) and preferably also or succinic acid. As a result of the further processing, the end residue solids may be dried to yield DDG or DDGS which is free flowing and more readily handled than are the similar products of other prior processes, due to the removal of the glycerol.

The first essential further processing step according to the invention is distillation of the fermented to produce ethanol. Such distillation may be by use of a stripping column capable of handling a solids containing stream. The bottoms or stillage is then centrifuged and the thin stillage processed further in a clarification step, removing the dispersed solids to obtain a (sparkling) clear liquid. Clarification is accomplished with a cross-flow microfiltration system containing ceramic or mineral membranes. In this process particles in the range of 0.1 - 10 »m, depending on the membrane selected, are separated from the thin stillage. High and stable fluxes may be obtained through computer controlled backflushing and proper membrane selection. These new microfiltration membranes are known as such and are commercially available from known suppliers. Such known apparatus may be incorporated into the overall apparatus which practices the processes of this invention.

By way of more specific example, the processing of a chemical clarification process may proceed by taking up to 20 percent of the stillage and liming it to a pH of 9.0 to 12.0 while at or near boiling. The remainder of the stillage is adjusted to a pH range of 4.5 to 7.5 with a suitable alkali at temperatures as high as practicable. The two portions are then mixed and a precipitate of salts forms, the separation of which is improved by the addition of polyelectrolytes, followed by centrifugation. Following microfiltration is a (partial) softening step, mainly for the reduction of the divalent cation levels of calcium and magnesium. This will prevent the plugging and fouling of the downstream ion exclusion resin by salt deposits of divalent cations due to potential process upsets which would considerably reduce the operating temperature. As the stillage is clarified, it may be passed to and through an evaporator to remove as much water as possible and effect as high a solids concentration as is practicable. Due to proper clarification, the overall heat transfer coefficient involved will be considerably improved over thin stillage which has not been clarified, while the fouling of heat transfer surfaces will also be minimized. The clarified and concentrated stillage is passed to an ion exclusion apparatus such as that available from Illinois Water Treatment Company (IWT) of Rockford, Illinois and which contains a suitable resin material such as IWT's SM-51-Na resin or the similar resin available from Dow Chemical as Dowex 50-WX8. As the material passes through the ion exclusion apparatus, glycerol is "retained" while other ionic constituents are passed into an effluent stream. Recovery efficiencies are in the range of from about 80% to about 98%, and the purity of the glycerol recovered is in a range of from about 80% to about 90%. The apparatus may be a simple or multiple column system used as a pulsed bed or a simulated moving bed. Recycling may be used to maintain or increase product purity and/or recovery efficiency. Condensate from any evaporators used in the apparatus treated in a mixed bed ion exchange may serve as desorbent, with desorbent to recovered glycerol ratios in the range of from about 10 to about 25. Such a column is in ionic equilibrium and requires no regeneration. Effluent from a glycerol recovery ion exclusion apparatus may be passed through a comparable apparatus for recovery of succinic acid. The dilute succinic acid product is concentrated in an evaporator and purified through crystallization. The resulting by-product stream of the ion exclusion step, before or after succinic acid recovery, is ideal for use as a backset in the fermentation. It is a "clean" stream, which will increase osmotic pressure levels as well as reduce process water requirements.

The glycerol stream recovered from the ion exclusion apparatus and process may be further purified in a mixed bed ion exchanger and then concentrated and purified to any desired grade. Such concentration and purification may, for example, be accomplished by the use of an energy efficient vacuum/steam multiple effect evaporator, and distillation and refining unit such as those available from G. Mazzoni SpA of Italy, with the more concentrated glycerol being deodorized, bleached, filtered and/or polished as desired.

As a final example, the production of glycerol and succinic acid without specific preparation of a stillage having enhanced levels of those constituents is believed valuable.

### Example 6

Stillage from a facility for the production of ethanol from wet miller's biomass was centrifuged and the thin stillage subjected to microfiltration in a ceramic membrane unit. The clear permeate was partially softened and then concentrated through evaporation to 73 weight percent solids and fed to an IWT Adsep system consisting of a single 7.6 cm (three inch) I.D. column with a bed height of 158.75 cm (62.25 in) of IWT SM-51-Na resin. The concentrate was fed at a rate of litres/Min/M² (2 GPM/square foot), with 20% feed pulse at 1.442 liters/pulse. The glycerol containing effluent was passed through an IWT mixed bed ion exchange unit to improve purity; then adjusted to pH 7.0 using NaOH; then, using Mazzoni equipment, concentrated by evaporation to 85 weight percent glycerol; and distilled and refined to a CP/USP grade glycerol of high quality. Constituents in the material at stages in the process were as summarized in the following table showing weight distributions.

| Constituent | Stillage | Clarified conc. stillage | Adsep effluent | Final product |
|---|---|---|---|---|
| Total solids | 7.37 | 4.883 | 1.13 | Trace |
| Protein | 2.21 | 0.79 | Trace | ----- |
| Carbohydrates | 1.19 | 0.73 | 0.03 | ----- |
| Fat | 0.07 | 0.003 | 0.001 | ----- |
| Ash | 0.84 | 0.61 | 0.05 | ----- |
| Lactic acid | 1.42 | 1.29 | 0.02 | Trace |
| Succinic acid | 0.09 | 0.08 | 0.004 | ----- |
| Other | 0.343 | 0.27 | 0.035 | Trace |
| Glycerol | 1.21 | 1.11 | 0.035 | Trace |
| Water | 92.63 | 3.18 | 5.27 | 0.004 |
| Total | 100.00 | 8.063 | 6.40 | 0.984 |
| Percent Glycerol | 1.21 | 13.77 | 15.47 | 99.59 |

## Claims

1. A process for manufacturing ethanol and recovering glycerol as a by-product thereof, comprising the steps of:
Preparing a biomass mash;
fermenting the mash with yeast;
distilling the fermented mash to produce ethanol and stillage containing glycerol;
clarifying the stillage; and
passing the clarified stillage through an ion exclusion material for separating glycerol from the other constituents of the clarified stillage,
characterised in that the stillage is clarified by subjecting it to a cross-flow microfiltration system, having ceramic or mineral membranes, which is able to separate particles in the size range 0.1 to 10 »m.

2. A process according to claim 1 which includes the further step of:
passing the effluent from the glycerol separation step through another ion exclusion material to recover succinic acid.

3. A process according to any preceding claim, wherein the step of clarifying further comprises centrifugally separating solids from liquid constituents prior to microfiltration.

4. A process according to any preceding claim wherein immobilised yeast cells are mixed with the biomass mash.

5. A process according to any preceding claim wherein yeast cells are mixed with the biomass mash in a concentration in excess of 300 x 10⁶per ml.

6. A process according to any preceding claim wherein a biomass mash is prepared with a dextrose equivalent of at least 30.

7. A process according to any preceding claim wherein the biomass mash is prepared having a reducing sugar concentration of at least 200 g/l.

8. A process according to any preceding claim wherein the pH of the mash is kept substantially constant during the first half of the fermentation process.

## Patentansprüche

1. Verfahren zur Herstellung von Ethanol und zur Gewinnung von Glycerin als Nebenprodukt desselben, das die folgenden Stufen umfaßt:
Herstellung einer Biomassen-Maische,
Fermentieren der Maische mit Hefe,
Destillieren der fermentierten Maische unter Bildung von Ethanol und Glycerin enthaltender Schlempe,
Klären der Schlempe und
Hindurchleiten der geklärten Schlempe durch ein Ionenausschlußmaterial zur Abtrennung des Glycerins von den anderen Bestandteilen der geklärten Schlempe,
dadurch gekennzeichnet, daß die Schlempe in der Weise geklärt wird, daß man sie in ein Querstrom-Mikrofiltrations-System, das Keramik- oder Mineral-Membranen aufweist, einführt, das in der Lage ist, Teilchen in dem Größenbereich von 0,1 bis 10 »m abzutrenne.

2. Verfahren nach Anspruch 1, das die folgende weitere Stufe umfaßt:
Hindurchleiten des Abstroms (Abwassers) aus der Glycerinabtrennungsstufe durch ein anderes Ionenausschlußmaterial zur Gewinnung von Bernsteinsäure.

3. Verfahren nach einem der vorhergehenden Ansprüche, bei dem die Klärungsstufe außerdem umfaßt die Zentrifugenabscheidung von Feststoffen von den flüssigen Bestandteilen vor der Mikrofiltration.

4. Verfahren nach einem der vorhergehenden Ansprüche, bei dem immobilisierte Hefezellen mit der Biomassen-Maische gemischt werden.

5. Verfahren nach einem der vorhergehenden Ansprüche, bei dem Hefezellen mit der Biomassen-Maische in einer Konzentration von mehr als 300 x 10⁶ pro ml gemischt werden.

6. Verfahren nach irgendeinem der vorhergehenden Ansprüche, bei dem eine Biomassen-Maische hergestellt wird mit einem Dextrose-Äquivalent von mindestens 30.

7. Verfahren nach irgendeinem der vorhergehenden Ansprüche, bei dem die Biomassen-Maische so hergestellt wird, daß sie eine Konzentration an reduzierendem Zucker von mindestens 200 g/l aufweist.

8. Verfahren nach irgendeinem der vorhergehenden Ansprüche, bei dem der pH-Wert der Maische während der ersten Hälfte der Fermentationsstufe im wesentlichen konstant gehalten wird.

## Revendications

1. Un procédé de production d'éthanol avec récupération de glycérol en tant que sous-produit, comprenant des étapes de :
préparation d'un broyat d'une biomasse ;
fermentation du broyat avec une levure ;
distillation du broyat fermenté pour produire de l'éthanol et une phase dormante contenant le glycérol ;
clarification de la phase dormante ; et
passage de la phase dormante par un matériel d'exclusion d'ion pour séparer le glycérol des autres constituants de la phase dormante clarifiée, caractérisé en ce que la phase dormante est clarifiée en la soumettant à un système de microfiltration tangentielle, comportant des membranes céramiques ou minérales, pouvant séparer des particules de 0,1 à 10 »m de granulométrie.

2. Un procédé selon la revendication 1, qui inclut en outre l'étape de :
passage de l'effluent de l'étape de séparation du glycérol par un autre matériel d'exclusion d'ion pour récupérer de l'acide succinique.

3. Un procédé selon l'une quelconque des revendications précédentes, dans lequel l'étape de clarification comprend en outre une séparation des solides des constituants liquides par centrifugation préalable à la microfiltration.

4. Un procédé selon l'une quelconque des revendications précédentes, dans lequel des cellules de levure immobilisées sont mélangées avec le broyat de biomasse.

5. Un procédé selon l'une quelconque des revendications précédentes, dans lequel les cellules de levure sont mélangées avec le broyat de biomasse dans une concentration en excès de 300 x 10⁶ par ml.

6. Un procédé selon l'une quelconque des revendications précédentes, dans lequel le broyat de biomasse est préparé avec un équivalent dextrose d'au moins 30.

7. Un procédé selon l'une quelconque des revendications précédentes, dans lequel le broyat de biomasse est préparé de manière à avoir une concentration en sucre réducteur d'au moins 200 g/l.

8. Un procédé selon l'une quelconque des revendications précédentes, dans lequel le pH du broyat est gardé sensiblement constant durant la première moitié du procédé de fermentation.
